# FASCICULE DE BREVET EUROPEEN

(11) **EP 0 487 441 B1**
(45) Date de publication et mention de la délivrance du brevet: **24.07.1996**
(21) Numéro de dépôt: 91440100.5
(22) Date de dépôt: 22.11.1991
(51) Int. Cl.: A61B 5/055, G01R 33/28, H05K 9/00

(54) **Moniteur de surveillance des paramètres physiologiques vitaux d'un patient en cours d'examen dit IRM**
Überwachung von lebenswichtigen, physiologischen Grössen während der Kernspin-Bild-Untersuchung eines Patienten
Monitoring vital physiological parameters of a patient during NMR imaging examination

(30) Priorité: 23.11.1990 FR 9014846
(43) Date de publication de la demande: 27.05.1992
(73) Titulaire: ODAM, S.A., F-67160 Wissembourg (FR)
(72) Inventeur: Muller, Gérard, 57410 - Rohrbach-les-Bitche (FR); Kraemer,Michel, 67360-Woerth (FR); Lanoux,Michel, 67160-Wissembourg (FR)
(74) Mandataire: Nuss, Pierre

(56) Documents cités:
- EP-A- 0 132 785
- EP-A- 0 173 130
- MAGNETIC RESONANCE IN MEDICINE vol.6, no. 2, Février 1988, DULUTH, MN (US) pages 240 -145; R. ROKEY ET AL.: 'Monitoring of Acutely Ill Patients during NMR Imaging...' Section "Equipments and Methods"

## Description

La présente invention se rapporte à un moniteur de surveillance des paramètres physiologiques vitaux d'un patient en cours d'examen par la technique de l'imagerie par résonance magnétique nucléaire.

On sait déjà réaliser des appareils autonomes capables de surveiller un paramètre ou un groupe de paramètres physiologiques d'un patient en cours d'examen médical.

L'examen mettant en oeuvre le principe connu de la résonance magnétique nucléaire des spins magnétiques des protons d'hydrogène présents dans les tissus, nécessite la création d'un milieu ou d'un environnement électromagnétique dans lequel est plongé l'objet ou le sujet à examiner.

Ici, et dans la suite du texte, on entendra par milieu ou environnement électromagnétique, l'ensemble de l'espace dans lequel les trois champs nécessaires conjuguent leurs effets de façon connue pour donner l'image de la zone explorée du sujet en cours d'examen, à savoir:
. le champ principal qui est un champ magnétique constant et homogène de forte intensité ;
. le gradient, qui est un champ magnétique variable en fonction de l'espace ;
. le champ magnétique oscillant à haute fréquence dans le domaine des radiofréquences.

Lors de l'examen, le patient est allongé sur une couchette et amené dans un espace en forme de tunnel appelé tunnel d'exploration constituant la zone centrale d'un aimant surpuissant généralement plongé dans un milieu cryogénique pour le rendre supraconducteur. Dans ce tunnel règne et se concentre l'environnement électromagnétique nécessaire à l'exploration.

Afin de ne pas perturber cet environnement électromagnétique, l'aimant, son bâti et tous ses dispositifs annexes proches sont placés dans une enceinte électriquement blindée, dont les parois ou cloisons sont réalisées en tôles de cuivre assemblées sans discontinuité électrique, formant cage de Faraday. Ensuite, les signaux d'exploitation et de commande sont filtrés par plusieurs batteries de filtres spécifiques et analysés par des circuits placés hors de l'enceinte puis visualisés sous forme d'image par un ou plusieurs écrans sur une console.

Les perturbations engendrées par un appareil de surveillance affectant le bon fonctionnement du matériel d'exploration IRM peuvent se regrouper en deux catégories distinctes :
. les parasites électromagnétiques de hautes fréquences perturbant l'action du champ magnétique oscillant. Pour cette unique raison tout appareil générant des parasites électromagnétiques est à proscrire dans l'enceinte blindée
. les déviations des lignes du champ principal à l'extérieur du tunnel d'exploration. Ces déviations proviennent de la présence d'une ou plusieurs masses ferromagnétiques et sont de nature à modifier l'uniformité nécessaire du champ principal régnant à l'intérieur de l'aimant. Il convient donc de veiller à ce que la somme des masses ferromagnétiques présentes dans l'appareil de surveillance n'excèdent pas une valeur limite fonction de la distance qui le sépare de l'aimant supraconducteur.

Inversement, l'appareil de surveillance placé à proximité du tunnel d'exploration subit des perturbations dues à l'environnement magnétique spécifique de l'installation IRM.

Ainsi, tout appareil dont le fonctionnement est susceptible d'être perturbé par l'environnement magnétique ne convient pas en l'état et nécessite des modifications importantes et des protections adaptées. Bien entendu, l'utilisation de tout support magnétique d'informations ou d'écrans à tubes cathodiques s'avère impossible.

Ainsi, aucun système informatique conventionnel complet ne peut convenir en raison de ses mémoires de masse et de ses supports ou composants magnétiques divers.

Il ne peut convenir non plus sans diverses protections adaptées car l'unité centrale génère sur le circuit électrique des signaux parasites.

De la même manière, l'électronique de commande de l'affichage nécessite des fréquences de fonctionnement élevées générant des parasites susceptibles de perturber le fonctionnement de l'installation IRM.

Ainsi, le bon fonctionnement d'un appareil de surveillance à proximité de l'installation IRM entraîne de nombreuses difficultés non encore maîtrisées à ce jour.

Un patient, tel qu'un accidenté ou un prématuré placé dans le tunnel d'exploration, nécessite une surveillance constante de la part d'un médecin ou d'un personnel qualifié, et la possibilité d'une intervention rapide en cas d'incident physiologique.

Or, l'étroitesse du tunnel d'exploration et sa longueur destinées à accueillir le corps entier, ne permettent pas à un personnel de surveillance de disposer d'un champ de vision suffisant pour observer convenablement le patient au cours de l'examen.

Par ailleurs, le champ magnétique particulièrement intense dans cette zone ne permet l'utilisation d'aucun ensemble traditionnel par exemple optique de surveillance mettant en oeuvre une ou plusieurs caméras vidéo.

Il faut ajouter que, bien entendu, ce type de surveillance ne serait pas suffisant pour renseigner sur l'état du patient et les soins immédiats à lui apporter en cas de défaillance momentanée.

En raison déjà de l'impossibilité de disposer de cette surveillance visuelle minimale, le besoin d'une surveillance générale constante par un automate revêt une importance encore plus grande.

Par ailleurs, un appareil de surveillance extérieur à l'enceinte blindée, même s'il était techniquement réalisable et opérationnel, ne présenterait pas d'intérêt en raison de la nécessité impérieuse, pour le personnel de surveillance, de se trouver à côté du patient pour intervenir immédiatement en cas de défaillance de celui-ci.

Le médecin de surveillance ne peut entrer dans l'enceinte blindée en cours d'exploration sous peine de fausser les mesures, et il n'est pas question, pour des raisons de rentabilité, d'affecter deux personnes à cette tâche.

Ainsi, cette impossibilité d'assurer convenablement une surveillance par un appareil à proximité du matériel d'exploitation IRM, non seulement ne cadre pas avec l'emploi des techniques modernes d'exploration, mais constitue un abandon difficilement admissible dans le cas de patients fragiles ou dans un état grave.

Aussi, la présence d'un appareil de surveillance à l'intérieur même de l'enceinte blindée s'impose-t-elle.

Ce besoin important, non encore satisfait à ce jour, a suscité des efforts répétés, soutenus et de grande ampleur de la part des inventeurs pour concevoir et mettre au point un automate de surveillance fonctionnant de façon satisfaisante à proximité du tunnel d'exploration.

Par le document "Magnetic Resonance in Medicine", vol. 6, n° 2, Février 1988, Duluth, pages 240-245, on connait déja un dispositif d'acquisition et de visualisation de l'électrocardiogramme d'un patient soumis à un examen par imagerie RMN.

Toutefois, ce document ne mentionne que la visualisation d'un seul paramètre physiologique, à savoir l'électrocardiogramme.

En outre, les signaux relevés sur le patient sont transmis par la voie d'ondes radiofréquences à l'extérieur de l'enceinte blindée en vue de leur traitement et de leur affichage sur différents moniteurs, dont un est disposé dis l'enceinte blindée contenant l'imageur RMN. Cette transmission de signaux radiofréquences est donc sujet à de nombreuses interférences avec les champs et les gradients magnétiques générés par l'imageur, ce qui entraine la génération de nombreux artefacts et compromet la fiabilité des informations affichées.

La présente invention a pour but de proposer un moniteur de surveillance pouvant fonctionner dans l'enceinte blindée contenant l'imageur et à proximité de l'aimant supraconducteur d'imagerie médicale par résonance magnétique nucléaire et ainsi de permettre au personnel de surveillance se tenant près du patient de disposer en permanence des informations sur son état, par la surveillance de plusieurs paramètres physiologiques, les signaux captés correspondants étant traités par le moniteur à l'intérieur même de l'enceinte blindée, sans transmission à l'extérieur.

A cet effet, l'invention a pour objet un moniteur de surveillance présentant les caractéristiques mentionnés dans la revendication 1.

Ainsi, l'appareil de surveillance selon l'invention permet simultanément pour la personne chargée de la surveillance et se tenant à côté du patient, de connaître son état et de pouvoir intervenir immédiatement en cas de défaillance ou de difficultés.

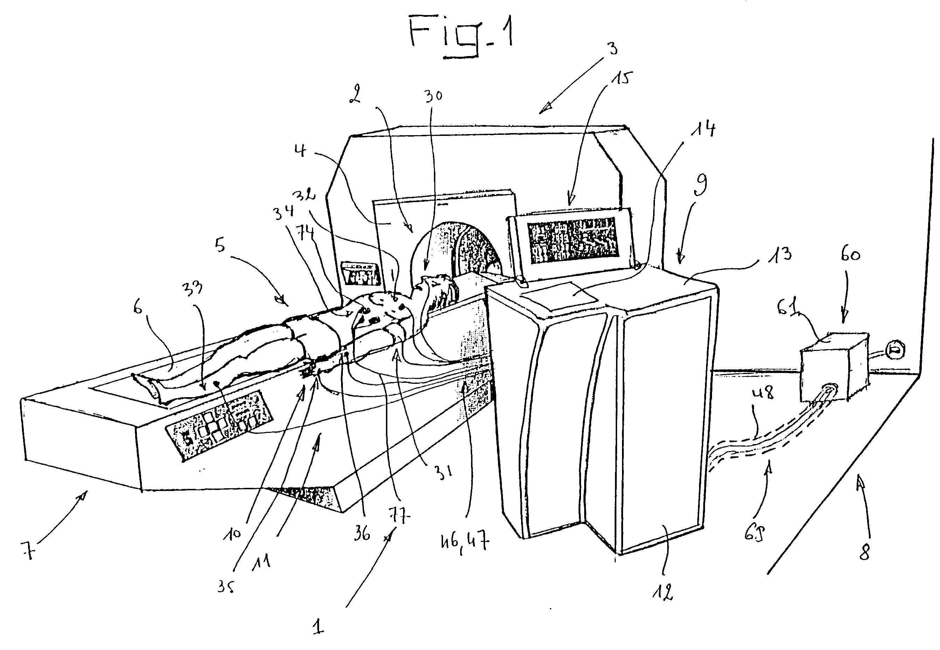

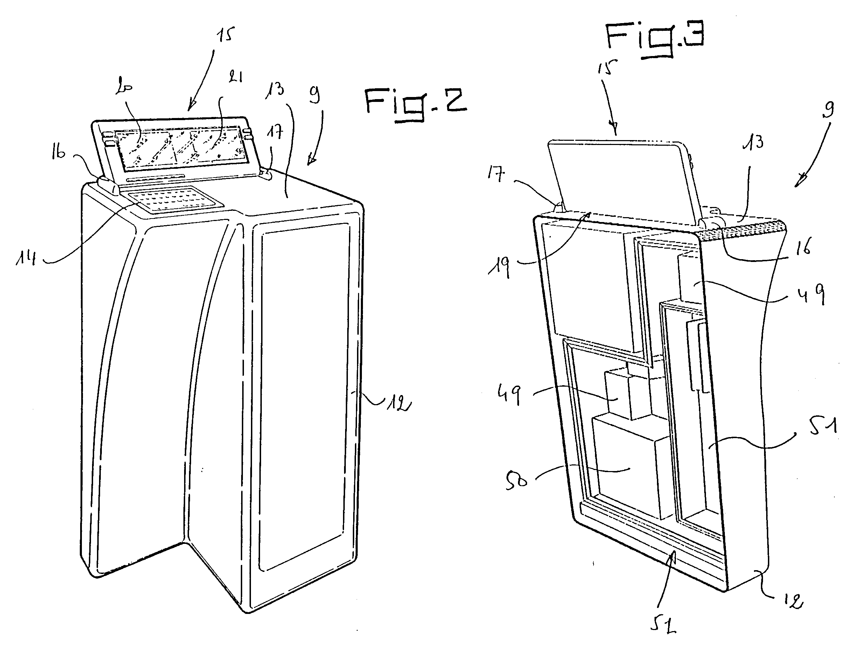

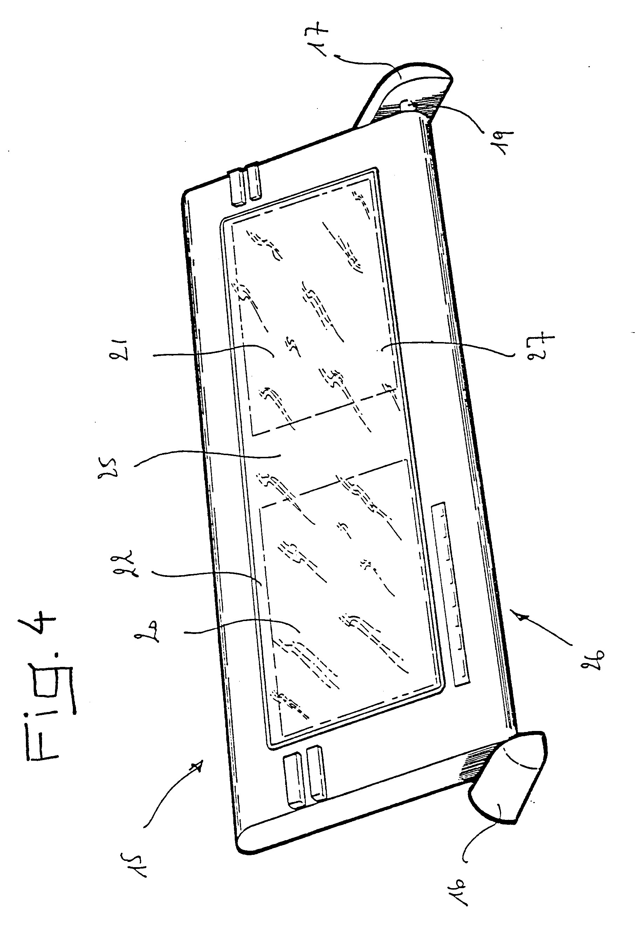

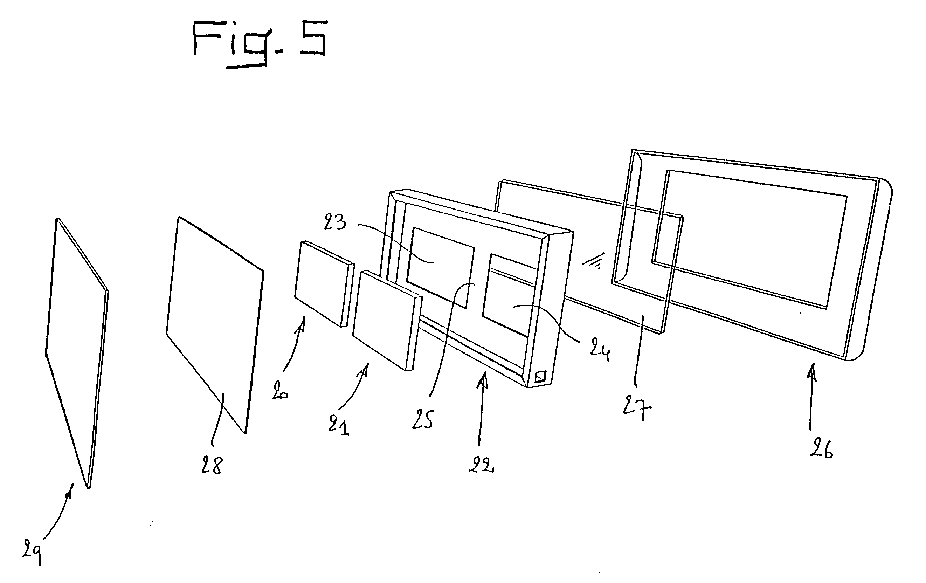

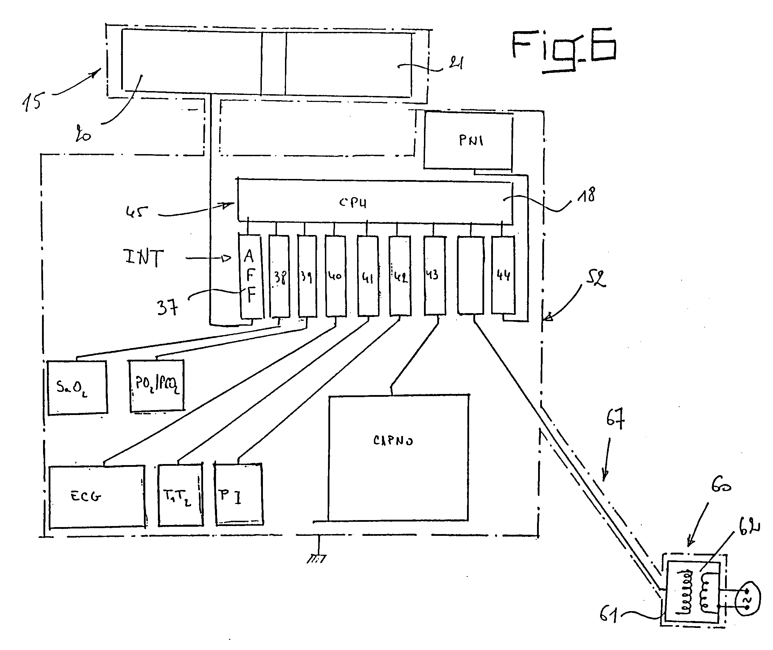

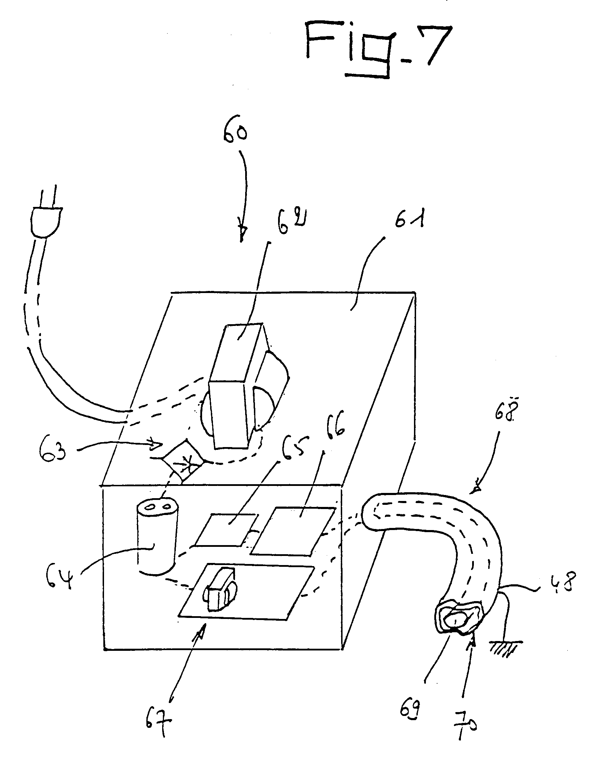

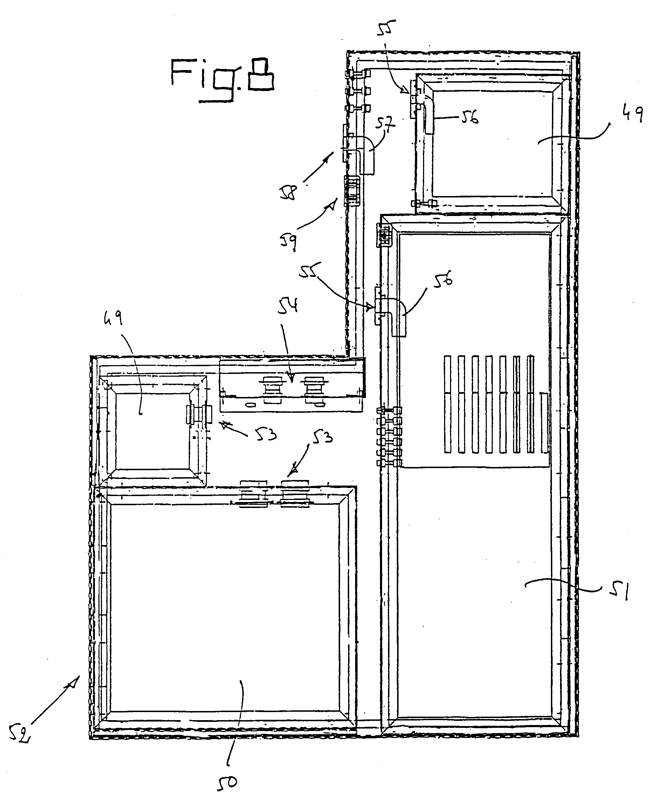

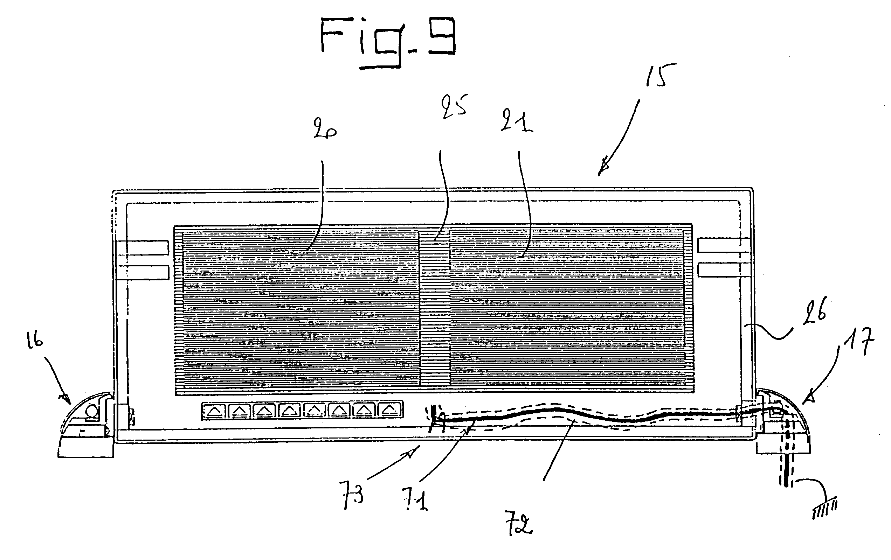

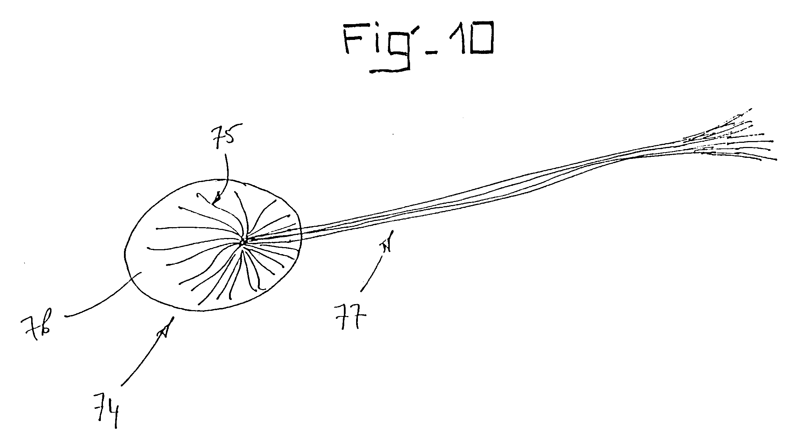

Les caractéristiques techniques détaillées et divers autres avantages sont contenues dans la description qui suit, effectuée à titre d'exemple non limitatif en référence aux dessins accompagnants dans lesquels :
. la figure 1 est une vue générale en perspective simplifiée du matériel d'exploration dit IRM et du moniteur selon l'invention disposé à proximité de celui-ci ;
. la figure 2 est une vue d'ensemble en perspective du moniteur selon l'invention vu de l'avant;
. la figure 3 est une vue d'ensemble en perspective du moniteur selon l'invention vu de l'arrière ;
. la figure 4 est une vue en perspective du panneau de visualisation ;
. la figure 5 est une vue en perspective dite " en éclatée" du panneau de visualisation ;
. la figure 6 est une vue schématique des différents blocs fonctionnels ;
. la figure 7 est une vue schématique de l'alimentation du moniteur montrant son emplacement par rapport au corps de l'appareil ;
. la figure 8 est une vue générale schématique en élévation des compartiments techniques illustrant la disposition des caissons d'isolation et leur sortie de ventilation ;
. la figure 9 est une vue en perspective simplifiée de la sortie du câble de liaison entre le corps de l'appareil et le panneau de visualisation ;
. la figure 10 est une vue schématique en plan d'une électrode d'électrocardiographie.

L'idée générale inventive consiste à concevoir et à construire un moniteur de surveillance d'un type entièrement nouveau de plusieurs paramètres physiologiques vitaux captés sur un patient, moniteur qui soit capable de fournir des mesures précises et fiables dans des conditions d'environnement magnétique intense existant à proximité immédiate d'une installation à Imageur à Résonnance Magnétique, ci-après dénommée IRM, et inversement de ne pas perturber par son fonctionnement les signaux détectés et analysés par cette installation IRM.

On a représenté sur la figure 1 l'implantation d'ensemble d'une installation IRM référencée 1 comprenant un imageur à résonance magnétique nucléaire qui se compose essentiellement d'une part d'un tunnel d'exploration 2 dans un bâti 3 abritant un électro-aimant surpuissant 4 en vue de l'examen d'un patient 5 placé de façon connue sur une couchette 6 prévue sur un support 7, dans une enceinte 8 blindée au plan électrique et étanche au rayonnement électromagnétique et d'autre part d'un pupitre de commande extérieur à l'enceinte blindée (non représentée). On place à proximité immédiate du tunnel de l'imageur un moniteur 9 de surveillance conforme à l'invention qui capte, analyse et surveille une pluralité de paramètres physiologiques vitaux prélevés sur le patient 5 placé dans le tunnel d'exploration 2 à l'aide d'un ensemble d'électrodes ou de capteurs tels que 10, adaptés aux types de paramètres surveillés reliés au moniteur 9 par une liaison multifilaire et multiconduits 11.

On décrira tout d'abord le moniteur 9 dans son ensemble, c'est-à-dire dans ses caractéristiques générales, en se reportant aux figures 1 à 3.

Le moniteur 9 est un appareil reposant sur le sol, en disposition verticale, équipé d'une carrosserie 12 à ouverture arrière, présentant sur le dessus un pupitre 13 de travail comportant un clavier de commande 14 pour communiquer avec l'opérateur et un panneau de visualisation 15 monté pivotant sur deux articulations 16 et 17 de part et d'autre du panneau entre une position rabattue sur le pupitre, qui est une position de transport, et une position ergonomique de travail dont l'inclinaison est appropriée pour faciliter la lecture. Des touches de menu préprogrammées de façon connue pour être correlées avec les messages apparaissant à l'écran sont, par exemple, placées en bas du panneau de visualisation.

A l'intérieur de la carrosserie et sous le pupitre 13 se trouve une unité centrale 18 de gestion et d'affichage qui gère les différents modules fonctionnels et commande l'affichage des paramètres surveillés sous forme alphanumérique et graphique sur le panneau de visualisation 15. Cette unité centrale est encore appelée unité de gestion-affichage en raison de sa fonction principale. Elle est également référencée CPU.

Comme représenté sur les figures 4 et 5, le panneau de visualisation 15 est monté pivotant autour d'un axe horizontal 19 sur la face supérieure de l'appareil.

Il est formé de deux écrans plats 20 et 21 séparés utilisant un matriçage de cellules électroluminescentes.

Ces écrans plans 20 et 21 sont montés juxtaposés dans un carter plan 22 en panneau formant châssis de blindage dans lequel ils sont convenablement immobilisés.

Ce carter comporte sur sa face avant deux ouvertures juxtaposées 23 et 24 vers l'amont correspondant à l'emplacement des écrans.

Ces ouvertures sont séparées par une bande transversale technique de recouvrement 25.

L'ensemble est maintenu dans un cadre-enveloppe 26 du type panneau maintenant contre les écrans deux vitres blindées 27 et 28 de façade avant et arrière.

Ces vitres sont transparentes, mais blindées c'est-à-dire opaques aux rayonnements électriques et électromagnétiques.

Ce blindage est constitué d'une feuille portant sur sa face interne une métallisation transparente reliée au carter 22.

L'ensemble est fermé sur la face arrière par une plaque obscure 29.

Ces différentes pièces constituant le panneau de visualisation sont représentées de façon indépendante sur la figure 5 et à l'état montées sur la figure 4.

Ainsi, les écrans sont enveloppés de toute part par une protection électrique et radioélectrique suffisante de l'intérieur vers l'extérieur et inversement pour former le panneau de visualisation et d'affichage tel que 15.

A titre d'exemple non limitatif le moniteur représenté comporte les modules de surveillance suivants schématisés sur la figure 6, à laquelle on se reportera plus particulièrement à présent :
. un module de surveillance de capnographie-respiration qui mesure les gaz expirés par le patient à l'aide d'un embout buccal ou nasal approprié 30, et ci-après référencé et appelé module CAPNO ;
. un module de surveillance de la pression artérielle par méthode non invasive du patient à l'aide d'un brassard 31 ci-après référencé et appelé module PNI ;
. un module de surveillance de températures prélevées en différents points du patient, par exemple en deux points, à l'aide de deux sondes de température 32 et 33 mesurant deux températures T1 et T2, ci-après référencé et dénommé module T1T2 ;
. un module de surveillance électrocardiographique ci-après référencé et appelé module ECG, dont les paramètres sont captés par des électrodes telles que 34 de recueil du signal d'électrocardiogramme ;
. un module de surveillance de la saturation en oxygène de l'hémoglobine, par exemple par le procédé transcutané, ci-après référencé et appelé module SAO₂, combiné à un capteur 35 correspondant au procédé utilisé ;
. un module de surveillance des pressions partielles de l'oxygène et du gaz carbonique dissous dans le sang, ci-après référencé et appelé module PO2/PCO2, combiné à un capteur spécifique 36 ;
. un module de surveillance de la pression artérielle invasive PI à l'aide d'un capteur en contact avec le sang du patient.

Chaque module ci-dessus assurant une fonction de surveillance connue en soi est associé aux autres modules et géré par une unité de fonctionnement d'ensemble associée à une carte d'affichage et à une carte d'alimentation.

En outre, le moniteur comporte une carte principale 18 de fonctionnement appelée carte de gestion-affichage, combinée à une carte d'affichage 37 désignée aussi par AFF et à des cartes interfaces référencées 38 à 44 et désignées aussi par INT appropriées aux fonctions des différents modules de surveillance, l'ensemble étant ci-après dénommé unité de gestion-affichage 45, dont la fonction essentielle consiste à gérer l'ensemble des modules, l'affichage des paramètres et l'interfaçage avec l'utilisateur et avec les modules.

Le moniteur comporte également un certain nombre de conducteurs internes reliant chaque module de mesure à l'unité de gestion-affichage 45, et la liaison multifilaire et multiconduits 11 rassemblant un nombre de conducteurs externes reliant chaque module de mesure à son ou à ses capteurs correspondants, référencés ci-dessous par des liaisons conductrices telles que 46 et des liaisons tubulaires 47.

Dans le but d'éviter les émissions électromagnétiques par les conducteurs externes pouvant constituer des antennes et internes de liaison et d'alimentation, émissions qui perturberaient l'environnement magnétique et électromagnétique et le fonctionnement de l'installation IRM, les caractéristiques suivantes de construction de l'appareil sont adoptées :
. tous les conducteurs sont entourés d'un blindage tel que 48 dont les extrémités sont reliées aux masses de l'appareil et de l'enceinte blindée ;
. les longueurs de conducteurs sont optimisées en vue de réduire au maximum les longueurs susceptibles de rayonner ;
. chaque module ou groupe de modules est enfermé dans un boîtier tel que 49, 50 ou 51, petit, moyen ou grand (figures 3 et 8) en cuivre ou en matériau non magnétique étanche aux rayonnements électromagnétiques, dont le but est de diminuer le rayonnement résiduel autour des boîtiers ; ces boîtiers sont superposés, fixés les uns aux autres ;
. l'ensemble des boîtiers est placé dans un caisson principal 52 en matériau non magnétique et de tenue mécanique suffisante et étanche également aux rayonnements électromagnétiques, par exemple en acier inoxydable, dans le but d'éviter le rayonnement résiduel à l'extérieur du moniteur et d'améliorer la protection de l'installation IRM ;
. l'ensemble des sorties des conducteurs de signaux électriques à travers chaque boîtier ou le caisson principal est filtré à l'aide de connecteurs filtrants, respectivement petits et grands tels que 53 et 54 (figure 8), disposés sur chacune des sorties ;
. le nombre des conducteurs est optimisé de façon à réduire le nombre de sorties, donc le nombre de connecteurs filtrants à utiliser et les risques de fuites de rayonnement et de transmission ;
. chaque boîtier 49, 50 et 51 et le caisson 52 présentent la forme générale d'une boîte avec une porte arrière verticale. L'étanchéité de chaque fermeture par rapport aux émissions électromagnétiques est réalisée par tout moyen approprié, par exemple par une tresse de blindage pincée entre la porte et le boîtier ou par des lamelles à effet ressort ;
. un passage de sortie tel que 55, sous la forme de coudes métalliques tels que 56 de tuyauterie, assure le passage des liaisons fluidiques et la communication avec l'extérieur de chaque boîtier, tout en protégeant l'intérieur d'un rayonnement direct. Un autre coude 57 constitue la sortie 58 du caisson principal 52.

Le premier but de la présence des boîtiers concerne le rayonnement résiduel qui doit être le plus faible possible dans le caisson principal, pour augmenter l'efficacité du filtrage des conducteurs sortant sur l'extérieur.

Un deuxième but de la présence des boîtiers est que, s'ils n'existaient pas, chaque conducteur sortant d'un module donné pourrait cumuler et émettre les perturbations créées par ce module, mais aussi transmettre celles provenant des autres modules, augmentant le champ résiduel dans le caisson principal 51 et rendre moins efficace le filtrage de sortie qui s'effectue par un connecteur général de sortie 59 au niveau de la sortie du caisson principal.

Simultanément et fondamentalement, le moniteur est conçu pour ne pas subir les perturbations de l'installation IRM, en particulier celles dues au champ magnétique très intense existant à sa proximité immédiate, mais décroissant en fonction de l'éloignement.

Le champ magnétique principal intense développé ayant une valeur constante, il est sans effet sur le fonctionnement des composants électroniques tels que les transistors, circuits intégrés, mais il empêche l'utilisation des mémoires de masse à support magnétique tels que disques durs, disquettes, bandes magnétiques ..., qui sont remplacées par des mémoires solides dans la carte de l'unité de gestion-affichage.

De plus, le comportement de chaque module dans l'environnement électromagnétique a dû être étudié individuellement.

Le moniteur est alimenté électriquement par une alimentation générale 60. Elle se présente sous la forme d'un bloc constituant un boîtier 61 renfermant les différents circuits et composants. Il s'agit d'un transformateur principal 62, d'un circuit de redressement 63 et d'un filtrage 64, d'un circuit de régulation 65 et d'un circuit général de commande 66 Marche/Arrêt et du transformateur 67 déplacé du module CAPNO. On choisit de déporter l'alimentation générale 60 à l'extérieur du caisson de l'appareil auquel elle est reliée par un câble d'alimentation et de commande 68, et à l'éloigner le plus possible de celui-ci tout en restant à l'intérieur de l'enceinte blindée (figure 1).

Le boîtier 61 de l'alimentation est relié à la masse générale de l'appareil et de l'enceinte blindée, ainsi que le blindage du câble de liaison 68.

Celui-ci est constitué d'une âme 69 formée d'une pluralité de conducteurs, entourée d'un blindage simple ou double 70 ou de plusieurs enveloppes successives blindées.

Si nécessaire, l'ensemble sera placé dans un profilé métallique, goulotte ou autre, relié à la masse générale.

Une autre disposition est prise encore au niveau de l'alimentation générale et du câble de liaison moniteur-alimentation 68.

En raison de l'éloignement, selon l'invention, de l'alimentation, la chute de tension dans le câble de liaison n'est pas négligeable au regard de la tension délivrée. Il s'avérait nécessaire d'augmenter en conséquence la tension continue d'alimentation et d'utiliser une alimentation en tension réglable spécifique au moniteur, délivrant une tension plus importante que celle des alimentations standards pour micro-ordinateurs.

Par ailleurs, la sortie des conducteurs de liaison entre le pupitre 13 du moniteur et son panneau de visualisation 15 pivotant ne doit pas être filtrée pour garder les signaux intacts.

Pour disposer quand même d'une protection, la solution spécifique adoptée consiste à ne pas rompre la continuité du blindage entre l'écran et le caisson principal 52 de façon à ce que les boîtiers et le caisson se comportent comme une cage de Faraday unique.

Dans ce but, les conducteurs de liaison pour les signaux d'alimentation, de balayage, d'analyse et de prise en compte du clavier 14 sont rassemblés dans un câble 71 qui passe dans l'un des axes des articulations 16 ou 17 du panneau de visualisation 15, dont le diamètre interne est prévu à cet effet. En outre, les conducteurs sont entourés d'une gaine de blindage souple telle que 72 en liaison électrique avec le caisson principal 52 du moniteur et avec le carter 22 du panneau de visualisation 15.

Pour résoudre le problème de résistance à la torsion des conducteurs de liaison dans l'axe d'articulation, le point fixe 73 de connexion conducteur-écran est placé au milieu du panneau de visualisation, de manière à laisser une longueur suffisante de conducteur libre en torsion.

Le comportement des capteurs dans l'environnement électromagnétique a également été étudié.

Ainsi, les électrodes habituelles d'électrocardiographie ont dû être remplacées, car les parties métalliques de l'électrode peuvent, dans cet environnement électromagnétique, provoquer des brûlures de la peau, et la liaison amovible entre l'électrode et le cordon de liaison avec le moniteur engendre des perturbations électromagnétiques. Ces électrodes ont été remplacées par des électrodes 74 formées de brins de fils 75 en carbone amorphe en disposition générale radiale sur un support plan autocollant 76, reliées électriquement au module correspondant par une liaison conductrice 77 constituée par la prolongation des brins de carbone formant l'électrode (figure 10).

Dans le cas où le gradient induit des parasites dans les fils de liaison moniteur-capteur entraînant la perturbation des signaux électriques transmis, et donc des courbes et valeurs affichées, une première solution consiste à changer la nature du signal transporté. Cette dernière solution est particulièrement adaptée au module SaO₂ pour lequel on choisit de transporter le signal lumineux du capteur par fibre optique jusqu'au module SaO₂ du moniteur, puis de le transformer en signal électrique à l'intérieur du boîtier moyennant des transducteurs adaptés.

Concernant la liaison électrique et fluidique entre les modules PNI et CAPNO et leurs capteurs correspondants, on remarquera qu'il s'agit de tubes dans lesquels circulent de l'air sous pression ou des échantillons de gaz, ils ne conduisent donc pas de signaux électriques. En conséquence, les sorties de ces tubes, d'une part au niveau des boîtiers de modules et d'autre part au niveau du caisson principal, ne nécessitent pas de connecteurs filtrants vis-à-vis des parasites électromagnétiques. Lesdites sorties sont cependant équipées des coudes métalliques précédemment cités par rapport au plan de sortie dans le but d'éviter la sortie en ligne droite des rayonnements résiduels électromagnétiques perpendiculaires au plan de sortie.

Il est bien entendu qu'au-delà des moyens décrits, diverses modifications évidentes et variantes simples entrent dans le cadre de la présente invention.

## Revendications

1. Moniteur de surveillance des paramètres physiologiques vitaux d'un patient en cours d'examen dans l'espace en tunnel d'un Imageur à Résonance Magnétique nucléaire dit IRM placé dans une enceinte blindée aux rayonnements électromagnétiques, moniteur composé de modules de surveillance associés à des capteurs correspondants par des conducteurs pour la mesure et la surveillance d'un paramètre ou d'un groupe de paramètres et d'un module de gestion-affichage pour la gestion de toutes les informations et la présentation des valeurs des paramètres sur un panneau de visualisation, caractérisé en ce que chaque module ou chaque groupe de modules est entouré d'un boîtier blindé, l'ensemble étant entouré d'un caisson blindé principal, de telle manière que le moniteur dans son ensemble et chacun de ses modules individuellement présentent un fonctionnement satisfaisant lorsque le moniteur est placé dans l'enceinte blindée contenant l'imageur IRM et à proximité de ce dernier, l'ensemble de gestion étant un système informatique sans aucun support magnétique.

2. Moniteur de surveillance selon la revendication 1, caractérisé en ce que :
. l'unité centrale de gestion-affichage comporte une carte de gestion-affichage dans laquelle les mémoires de masse sont remplacées par des mémoires solides ;
. chaque module de surveillance est constitué à partir d'une carte de fonction d'un appareil de surveillance autonome.

3. Moniteur de surveillance selon l'une des revendications précédentes, caractérisé en ce que le nombre et la longueur des conducteurs et le nombre des sorties sont optimisés.

4. Moniteur de surveillance selon l'une des revendications précédentes, caractérisé en ce que le boîtier d'alimentation est d'une part déporté par rapport au moniteur par un câble blindé relié au caisson, d'autre part délivre une tension réglable supérieure à la tension nécessaire pour tenir compte des pertes en ligne, le régulateur restant dans le boîtier principal.

5. Moniteur de surveillance selon l'une des revendications précédentes, caractérisé en ce que les sorties électriques des boîtiers d'une part, les sorties électriques du caisson principal vers les capteurs d'autre part, sont équipées de connecteurs filtrants.

6. Moniteur de surveillance selon l'une des revendications précédentes, caractérisé en ce que le câble de liaison avec le panneau de visualisation est un conducteur souple à blindage dont les extrémités sont reliées aux masses et en ce qu'il passe à l'intérieur d'une articulation.

7. Moniteur de surveillance selon l'une des revendications précédentes, caractérisé en ce que les sorties des tubes de gaz sont équipées de tubulures coudées à 90° d'angle par rapport au plan de la sortie.

## Claims

1. Control monitor of the vital physiological parameters of a patient during examination in the tunnel space of a nuclear magnetic resonance imager, known as MRI, placed in a chamber shielded from electromagnetic radiation, the monitor being composed of control modules associated with corresponding sensors by conductors for the measurement and control of a parameter or a group of parameters and of a management display module for the management of all information and the presentation of the values of the parameters on a display panel, characterized in that each module or each group of modules is surrounded by a shielded box, the unit being surrounded by a main shielded housing in such a way that the monitor in its entirety and each of its modules individually operates satisfactorily when the monitor is placed in the shielded chamber containing the MRI imager and in the vicinity thereof, the management unit being a computer system without any magnetic support.

2. Control monitor according to Claim 1, characterized in that the central management display unit comprises a management display card in which the bulk memories are replaced by solid memories; each control module consists of a function card of an autonomous control apparatus.

3. Control monitor according to one of the preceding claims, characterized in that the number and length of the conductors and the number of outputs are optimized.

4. Control monitor according to one of the preceding claims, characterized in that the supply box is on the one hand offset relative to the monitor by a shielded cable connected to the housing and on the other hand delivers an adjustable voltage higher than the voltage required to allow for line losses, the regulator remaining in the main box.

5. Control monitor according to one of the preceding claims, characterized in that the electrical outlets of the boxes on the one hand and the electrical outlets of the main housing toward the sensors on the other hand are equipped with filtering connectors.

6. Control monitor according to one of the preceding claims, characterized in that the connecting cable to the display panel is a shielded flexible conductor of which the ends are connected to the earths and in that it passes inside a coupling.

7. Control monitor according to one of the preceding claims, characterized in that the gas tube outlets are equipped with manifolds bent at angles of 90° to the plane of the outlet.

## Patentansprüche

1. Monitor zur Überwachung von lebenswichtigen physiologischen Parametern eines Patienten während der Untersuchung im Tunnelbereich eines Kernspinresonanz-Bildgerätes, sog. IRM, das in einem gegen elektromagnetische Strahlungen abgeschirmten Raum steht, wobei der Monitor aus Überwachungsmodulen, die mit entsprechenden Aufnehmern durch Leiter zur Messung und Überwachung eines Parameters oder einer Gruppe von Parametern verbunden sind und einem Steuerung-Anzeige-Modul zur Steuerung aller Informationen und Präsentation der Parameterwerte auf einer Visualisierungstafel zusammengesetzt ist, dadurch gekennzeichnet, daß jedes Modul oder jede Gruppe von Modulen von einem abgeschirmten Gehäuse umhüllt ist, wobei die Anordnung von einem abgeschirmten Hauptkasten umhüllt ist, derart, daß der Monitor in seiner Anordnung und jedes seiner Module individuell eine ausreichende Funktionsfähigkeit aufweisen, wenn der Monitor in dem das IRM-Bildgerät enthaltenden abgeschirmten Raum und in der Nähe von letzterem angeordnet ist, wobei die Steuerungsanordnung ein Informatiksystem ohne jeden magnetischen Träger ist.

2. Überwachungsmonitor nach Anspruch 1, dadurch gekennzeichnet, daß
- die Steuerung-Anzeige-Zentraleinheit eine Steuerung-Anzeige-Karte aufweist, auf der die Massenspeicher durch Festkörperspeicher ersetzt sind;
- jedes Überwachungsmodul aus einer Karte mit der Funktion eines autonomen Überwachungsgerätes gebildet ist.

3. Überwachungsmonitor nach einem der vorhergehenden Ansprüche, dadurch gekennzeichnet, daß die Anzahl und Länge der Leiter und die Anzahl der Ausgänge optimiert sind.

4. Überwachungsmonitor nach einem der vorhergehenden Ansprüche, dadurch gekennzeichnet, daß das Versorgungsgehäuse einerseits bezüglich des Monitors durch ein mit dem Kasten verbundenes, abgeschirmtes Kabel abgesetzt ist, andererseits eine steuerbare Spannung liefert, die größer ist als die Spannung, die nötig ist, um Leitungsverluste zu berücksichtigen, wobei der Regler im Hauptgehäuse bleibt.

5. Überwachungsmonitor nach einem der vorhergehenden Ansprüche, dadurch gekennzeichnet, daß die elektrischen Ausgänge der Gehäuse einerseits, die elektrischen Ausgänge des Hauptkastens zu den Aufnehmern andererseits mit filternden Verbindern ausgestattet sind.

6. Überwachungsmonitor nach einem der vorhergehenden Ansprüche, dadurch gekennzeichnet, daß das Kabel zur Verbindung mit der Visualisierungstafel ein elastischer Leiter mit Abschirmung ist, dessen Enden mit den Massen verbunden sind, und daß es ins Innere eines Gelenks reicht.

7. Überwachungsmonitor nach einem der vorhergehenden Ansprüche, dadurch gekennzeichnet, daß die Ausgänge der Gasröhren mit Stutzen ausgestattet sind, die im Winkel von 90° bezüglich der Ausgangsebene gebogen sind.
